# EUROPÄISCHE PATENTANMELDUNG

(11) **EP 4 749 633 A1**
(43) Veröffentlichungstag der Anmeldung: **27.05.2026**
(21) Anmeldenummer: 25216325.8
(22) Anmeldetag: 17.11.2025
(51) Int. Cl.: G16H 15/00, G16H 30/40, G16H 50/70

(54) **VERFAHREN UND VORRICHTUNG ZUR DOKUMENTATION VON PFLEGEMASSNAHMEN**

(30) Priorität: 21.11.2024 DE 102024134273
(71) Anmelder: FORBENCAP GmbH, 87527 Sonthofen (DE)
(72) Erfinder: Conle, Robert Ludwig, 87527 Sonthofen (DE)
(74) Vertreter: advotec.

(57) **Zusammenfassung**

Verfahren zur automatisierten Dokumentation von Pflegemaßnahmen zur Pflege eines Patienten, aufweisend die Schritte: Erfassen (S1) von Bilddaten von Interaktionen zwischen einer Pflegekraft und einem Patienten unter Verwendung einer Bildaufnahmeeinheit, Verarbeiten (S2) der erfassten Bilddaten unter Verwendung eines Bildverarbeitungsalgorithmus zum Erkennen der Interaktionen in den Bilddaten, und Generieren (S3) von Text- und/oder Sprachlabels zu den erkannten Interaktionen unter Verwendung eines maschinellen Labelgenerierung-Lernmodells zur automatisierten Dokumentation von Pflegemaßnahmen unter Verwendung der generierten Text- und/oder Sprachlabels.

## Beschreibung

Die Erfindung betrifft ein Verfahren und eine Vorrichtung zur automatisierten Dokumentation von Pflegemaßnahmen zur Pflege eines Patienten.

### Stand der Technik

Die Pflege von Patienten, insbesondere in der Alten- und Krankenpflege, erfordert eine sorgfältige Dokumentation der durchgeführten Pflegemaßnahmen. Diese Dokumentation dient dazu, die Qualität und Nachvollziehbarkeit der Pflege sicherzustellen sowie die Pflegekraft und den Patienten rechtlich abzusichern.

In der Praxis erfolgt die Dokumentation häufig manuell, was zeitaufwendig ist und wertvolle Ressourcen der Pflegekräfte beansprucht. Darüber hinaus werden digitale Ansätze unter Verwendung von Tablets usw. zur Pflege-Dokumentation zunehmend genutzt, allerdings erfordern auch diese Ansätze weiterhin eine signifikante menschliche Interaktion.

Die Notwendigkeit einer effizienten und präzisen Dokumentation ist insbesondere in Hinblick auf den steigenden Bedarf an Pflegekräften sowie die bestehende Personalknappheit von großer Bedeutung. Eine Reduzierung des Arbeitsaufwands für die Pflegekräfte könnte dazu beitragen, den Fokus verstärkt auf die eigentliche Pflege zu legen.

Es ist eine Aufgabe der Erfindung, ein Verfahren und/oder eine Vorrichtung zur automatisierten Dokumentation von Pflegemaßnahmen zur Pflege eines Patienten anzugeben.

### Offenbarung der Erfindung

Die Aufgabe wird gelöst durch ein Verfahren gemäß den Merkmalen des Patentanspruchs 1. Die Aufgabe wird gelöst durch eine Vorrichtung gemäß den Merkmalen des Patentanspruchs 10.

Gemäß einem bevorzugten Aspekt wird ein Verfahren zur automatisierten Dokumentation von Pflegemaßnahmen zur Pflege eines Patienten vorgeschlagen, umfassend die Schritte: Erfassen von Bilddaten von Interaktionen zwischen einer Pflegekraft und einem Patienten unter Verwendung einer Bildaufnahmeeinheit, Verarbeiten der erfassten Bilddaten unter Verwendung eines Bildverarbeitungsalgorithmus zum Erkennen der Interaktionen in den Bilddaten, und Generieren von Text- und/oder Sprachlabels zu den erkannten Interaktionen unter Verwendung eines maschinellen Labelgenerierung-Lernmodells (vorliegend auch als maschinelles Lernmodell oder auch kurz Modell bezeichnet), insbesondere eines Transformer-Modells.

Das Verfahren sieht vor, dass Bilddaten von Interaktionen zwischen einer Pflegekraft und einem Patienten erfasst werden. Diese Bilddaten dokumentieren vorzugsweise typische Pflegemaßnahmen wie Mobilisation, Körperpflege, Medikamentengabe und/oder Wundversorgung. Die Bildaufnahmeeinheit kann dabei in Form einer tragbaren Kamera, beispielsweise integriert in einer Smart-Brille, oder als stationär im Raum angeordnetes Gerät ausgeführt sein. Alternativ könnte die Bildaufnahmeeinheit auch Tiefenkameras oder multispektrale Kameras umfassen, um zusätzliche Details der Interaktionen zu erfassen.

Weiterhin erfolgt die Verarbeitung der erfassten Bilddaten, wobei ein Bildverarbeitungsalgorithmus eingesetzt wird, um die in den Bilddaten enthaltenen Interaktionen zu erkennen. Ein solcher Algorithmus kann beispielsweise auf Verfahren wie Segmentierung, Objekterkennung oder Pose-Estimation basieren. Zusätzlich oder alternativ könnten Ansätze zur Bewegungsanalyse, Aktivitätserkennung oder Anomalieerkennung integriert werden, um komplexe Pflegemaßnahmen zu identifizieren oder kritische Situationen zu detektieren.

In einem weiteren Schritt des Verfahrens werden aus den erkannten Interaktionen Text- und/oder Sprachlabels generiert. Dies erfolgt durch ein maschinelles Lernmodell, vorzugsweise ein Transformer-Modell, das zuvor anhand annotierter Pflegeprotokolle und Bilddaten trainiert wurde. Die Labels beschreiben vorzugsweise die durchgeführten Pflegemaßnahmen präzise und, insbesondere in zeitlicher Abfolge, strukturiert und können als Text und/oder in Form von Audio ausgegeben werden. Alternativ oder ergänzend könnten auch andere Lernmodelle, wie Recurrent Neural Networks (RNNs) oder Convolutional Neural Networks (CNNs), eingesetzt werden, um spezifische Aufgaben wie die Analyse zeitlicher Bildsequenzen zu bewältigen.

Das vorgeschlagene Verfahren bietet zahlreiche technische Vorteile. Es reduziert den manuellen Aufwand für die Pflegedokumentation, was den Pflegekräften mehr Zeit für die direkte Patientenbetreuung gibt. Durch den Einsatz maschineller Lernmodelle wird eine hohe Präzision und Konsistenz bei der Dokumentation gewährleistet, und die automatisch generierten Daten können vorzugsweise revisionssicher gespeichert und bei Bedarf überprüft werden. Die Flexibilität des Verfahrens ermöglicht vorzugsweise eine Anpassung an verschiedene Pflegeumgebungen und/oder -szenarien, während die Skalierbarkeit eine Implementierung in Einrichtungen unterschiedlichster Größe, von Pflegeheimen bis zu Krankenhäusern, unterstützt. Darüber hinaus stellt die Möglichkeit zur Anonymisierung der Daten sicher, dass datenschutzrechtliche Anforderungen eingehalten werden.

Es versteht sich, dass die erfindungsgemäßen Schritte sowie weitere optionale Schritte nicht notwendigerweise in der aufgezeigten Reihenfolge ausgeführt werden müssen, sondern auch in einer anderen Reihenfolge ausgeführt werden können. Ferner können weitere Zwischenschritte vorgesehen sein. Die einzelnen Schritte können zudem einen oder mehrere Unterschritte umfassen, ohne dass hierdurch der Umfang des erfindungsgemäßen Verfahrens verlassen wird.

Das maschinelle Lernmodell generiert vorzugsweise Labels automatisch aus Bilddaten, indem es während des Trainings gelernt hat, visuelle Muster, Bewegungen und Kontextinformationen mit spezifischen Bedeutungen zu verknüpfen. Dieser Prozess erfolgt vorzugsweise in mehreren Schritten, die ineinandergreifen. Zunächst könnten die erfassten Bilddaten vorverarbeitet werden, um sie für das Modell konsistent und leichter verarbeitbar zu machen. Dazu gehört vorzugsweise die Anpassung der Bildgröße, die Normalisierung von Farbräumen und die Entfernung von Bildrauschen. Für Videodaten könnten vorzugsweise Sequenzen von Frames extrahiert werden, um zeitliche Abläufe zu analysieren. Das maschinelle Lernmodell weist vorzugsweise ein Sprachmodell, insbesondere ein gro-ßes Sprachmodell, auf.

Das maschinelle Lernmodell kann ein GPT (Generative Pre-trained Transformer) aufweisen, das auf der Transformer-Architektur basiert und besonders gut für die Verarbeitung von Texten und die Generierung von natürlich klingender Sprache geeignet ist. Das maschinelle Lernmodell kann auch ein BERT (Bidirectional Encoder Representations from Transformers) aufweisen, das ebenfalls auf der Transformer-Architektur basiert und sich durch seine Fähigkeit auszeichnet, kontextuelle Zusammenhänge in Texten bidirektional zu analysieren. Alternativ kann das maschinelle Lernmodell ein T5 (Text-to-Text Transfer Transformer) aufweisen, das ebenfalls eine Transformer-Architektur verwendet und sich darauf spezialisiert hat, jede Textverarbeitungsaufgabe als Text-zu-Text-Problem zu formulieren.

Weiterhin kann das maschinelle Lernmodell ein XLNet aufweisen, das auf einer Transformer-Architektur mit autoregressiven und autoencoderartigen Mechanismen basiert und kontextuelle Abhängigkeiten besser modellieren kann. Ein weiteres mögliches Modell kann RoBERTa (Robustly Optimized BERT Approach) aufweisen, eine optimierte Version von BERT, die durch umfangreicheres Training auf größeren Datensätzen eine höhere Leistungsfähigkeit erreicht. Das Modell könnte auch ein ALBERT (A Lite BERT) sein, eine leichte und optimierte Variante von BERT, die weniger Speicherplatz benötigt und schneller trainierbar ist.

Darüber hinaus kann das maschinelle Lernmodell ein OpenAI Codex aufweisen, ein Transformer-basiertes Modell, das speziell für die Verarbeitung und Generierung von Code trainiert wurde. Für multimodale Anwendungen kann ein CLIP (Contrastive Language-Image Pre-training) Modell eingesetzt werden, das auf einer Transformer-Architektur basiert und Text- und Bildinformationen kombiniert, um visuelle und sprachliche Eingaben miteinander in Beziehung zu setzen. Schließlich könnte ein Transformer-XL verwendet werden, eine erweiterte Transformer-Architektur, die längere kontextuelle Abhängigkeiten modellieren kann.

Das Modell extrahiert vorzugsweise visuelle Merkmale aus den Daten. In den ersten Schichten eines neuronalen Netzwerks, beispielsweise in Convolutional Neural Networks (CNNs) oder Transformer-Modellen, könnten grundlegende Muster wie Kanten, Farben oder Texturen erkannt werden. Fortgeschrittene Schichten abstrahieren vorzugsweise diese Merkmale weiter und identifizieren komplexere Strukturen wie Objekte oder spezifische Handlungen, etwa das Anreichen eines Pflegeutensils oder das Waschen eines Patienten.

Mithilfe von Objekterkennungs- und Szenenanalyse-Algorithmen wie YOLO oder Mask R-CNN könnten vorzugsweise einzelne Objekte in den Bildern oder Videos erkannt und segmentiert werden. Diese Analyse ermöglicht es vorzugsweise, spezifische Pflegehandlungen wie die Vorbereitung einer Spritze oder das Umlagern eines Patienten in ihrem visuellen Kontext zu erkennen. Für dynamische Szenen, in denen Bewegungen entscheidend sind, könnte das Modell Bewegungen über mehrere Frames hinweg analysieren. Dabei könnten vorzugsweise optische Flussalgorithmen oder 3D-CNNs genutzt werden, um Aktivitäten wie das Aufrichten eines Patienten oder das Anlegen eines Verbands zu identifizieren.

Um die Informationen sinnvoll zu verknüpfen, interpretiert das Modell vorzugsweise die erkannten Muster und Bewegungen im Kontext der Pflegehandlung. Transformer-Modelle oder Recurrent Neural Networks (RNNs) könnten vorzugsweise eingesetzt werden, da sie zeitliche und räumliche Zusammenhänge berücksichtigen. So könnte das Modell erkennen, ob die Pflegekraft gerade einem Patienten ein Glas Wasser reicht, einen Blutdruck misst, einen Verbandswechsel vornimmt etc. oder ob sie eine andere Handlung ausführt. Diese kontextuelle Interpretation ist vorzugsweise entscheidend, um präzise Labels zu generieren.

Im letzten Schritt könnten die Ergebnisse der Objekterkennung, Bewegungsanalyse und Kontextinterpretation vorzugsweise mit zuvor erlernten Pflegeprotokollen abgeglichen werden. Das Modell könnte basierend auf dieser Analyse Labels erstellen, die die erkannte Handlung oder Beobachtung beschreiben, wie etwa "Patient gewaschen", "Medikament verabreicht" oder "Patient mobilisiert".

Das Modell kann diese Labels vorzugsweise automatisch generieren, weil es auf annotierten Datensätzen trainiert wurde, die Bilder und Videos mit präzisen Beschreibungen enthalten. Während des Trainingsprozesses könnte es lernen, wie spezifische visuelle Muster, wie etwa die Bewegung einer Hand oder die Position eines Pflegeutensils, mit bestimmten Pflegehandlungen korrelieren. Darüber hinaus könnten Transformer-Modelle vorzugsweise Informationen aus mehreren Quellen kombinieren, etwa aus Bilddaten, Bewegungsmustern und Umgebungsinformationen, um eine kontextuelle Interpretation der Szene zu ermöglichen.

Ein praktisches Beispiel für die automatische Labelgenerierung könnte vorzugsweise die Vorbereitung einer Spritze sein. Eine Kamera könnte aufzeichnen, wie eine Pflegekraft eine Spritze vorbereitet. Das Modell könnte dabei die Pflegekraft, die Spritze und die Bewegungssequenz erkennen und vorzugsweise das Label "Medikamentenspritze vorbereitet" generieren. In einem anderen Szenario könnte das Modell die Bewegungen der Pflegekraft und des Patienten während der Mobilisation analysieren und vorzugsweise das Label "Patient aus Bett gehoben" erzeugen. Auch bei der Kontrolle der Vitalzeichen eines Patienten könnte das Modell automatisch das Pflegeutensil, die Position der Pflegekraft und die Interaktion erkennen, um vorzugsweise das Label "Blutdruck gemessen" zu erstellen.

In einem weiteren bevorzugten Aspekt wird eine Vorrichtung zur automatisierten Dokumentation von Pflegemaßnahmen zur Pflege eines Patienten vorgeschlagen, wobei die Vorrichtung eine Auswerte- und Recheneinrichtung aufweist, die dazu ausgebildet ist, die folgenden Schritte auszuführen: Erfassen von Bilddaten von Interaktionen zwischen einer Pflegekraft und einem Patienten unter Verwendung einer Bildaufnahmeeinheit; Verarbeiten der erfassten Bilddaten unter Verwendung eines Bildverarbeitungsalgorithmus zum Erkennen der Interaktionen in den Bilddaten; und Generieren von Text- und/oder Sprachlabels zu den erkannten Interaktionen unter Verwendung eines maschinellen Lernmodells, insbesondere eines Transformer-Modells.

Die für das Verfahren gemachten Ausführungen gelten für die Vorrichtung entsprechend. Dabei versteht es sich, dass sprachliche Abwandlungen von verfahrensmäßig formulierten Merkmalen nach sprachüblicher Praxis für die Vorrichtung umformulierbar sind, ohne dass derartige Formulierungen explizit hier aufgeführt werden müssen.

In einem weiteren Aspekt wird vorgeschlagen, dass das Verfahren eine Bildaufnahmeeinheit umfasst, die eine Kamera und/oder eine Smart-Brille und/oder ein anderes tragbares Gerät zur Bilderfassung aufweist.

Die Bildaufnahmeeinheit ermöglicht vorzugsweise die Erfassung visueller Daten der Interaktionen zwischen Pflegekraft und Patient. Dabei kann die Kamera stationär oder mobil sein, während tragbare Geräte wie Smart-Brillen die Flexibilität der Pflegekräfte erhöhen. Alternativ oder ergänzend könnten auch andere tragbare Geräte wie Körperkameras oder Wearables mit integrierter Kamera verwendet werden. Diese Merkmale erleichtern vorzugsweise die nahtlose Integration in die Arbeitsabläufe der Pflegekraft, ohne die Pflegehandlung zu behindern. Die Verwendung tragbarer Bildaufnahmeeinheiten ermöglicht die Erfassung von Bilddaten in direkter Nähe der Pflegehandlung, was die Präzision der Dokumentation erhöht. Smart-Brillen können zusätzlich Kontextinformationen bereitstellen und die Blickrichtung der Pflegekraft berücksichtigen. Die Flexibilität bei der Auswahl der Bildaufnahmeeinheit trägt zur Anpassungsfähigkeit des Verfahrens an unterschiedliche Pflegeumgebungen bei. Tragbare Geräte minimieren den Platzbedarf und maximieren die Mobilität.

In einem weiteren Aspekt wird vorgeschlagen, dass das Verfahren eine Bildaufnahmeeinheit umfasst, die insbesondere stationär oder beweglich, in einem Raum, in dem der Patient gepflegt wird, und/oder an einem Körper der Pflegekraft und/oder an einem Körper des Patienten angeordnet ist.

Die Anordnung der Bildaufnahmeeinheit bestimmt vorzugsweise die Perspektive und Reichweite der erfassten Daten. Stationäre Einheiten sind in Räumen wie Patientenzimmern oder Pflegebereichen fixiert und ermöglichen eine kontinuierliche Überwachung. Bewegliche Bildaufnahmeeinheiten wie tragbare Kameras können vorzugsweise flexibel positioniert werden. Alternativ oder ergänzend könnten Sensoren direkt am Körper des Patienten oder der Pflegekraft angebracht werden, um eine personalisierte Erfassung zu gewährleisten. Stationäre Einheiten bieten eine dauerhafte, raumübergreifende Erfassung, während tragbare Geräte spezifische Details aus der Nähe dokumentieren. Körpergebundene Kameras liefern individuelle Perspektiven und verringern den Einfluss von Umgebungselementen. Die Vielseitigkeit der Anordnungsmöglichkeiten erhöht vorzugsweise die Funktionalität in verschiedenen Pflegeumgebungen. Bewegliche Bilderfassungseinheiten verbessern die Einsatzfähigkeit bei wechselnden Szenarien.

In einem weiteren Aspekt wird vorgeschlagen, dass der Bildverarbeitungsalgorithmus eine Segmentierung der Bilddaten und/oder eine Objekterkennung und/oder eine Bewegungsanalyse und/oder eine Aktivitätserkennung und/oder eine Gesichtserkennung und/oder eine Anomalieerkennung und/oder eine Objektverfolgung und/oder eine Klassifikation von Handlungen und/oder eine multimodale Bildverarbeitung und/oder eine Feature-Extraktion aufweist.

Der Bildverarbeitungsalgorithmus analysiert vorzugsweise die erfassten Bilddaten und extrahiert spezifische Informationen. Segmentierung unterteilt das Bild vorzugsweise in relevante Bereiche, während Objekterkennung bestimmte Pflegeutensilien oder Körperteile identifiziert. Bewegungs- und Aktivitätserkennung dienen vorzugsweise der Analyse dynamischer Szenen, während Gesichtserkennung den Patienten identifizieren oder Emotionen erfassen kann. Multimodale Bildverarbeitung kombiniert vorzugsweise verschiedene Datentypen, wie RGB- und Tiefendaten, für eine umfassendere Analyse. Diese Algorithmen ermöglichen vorzugsweise eine präzise Erkennung von Pflegehandlungen und verbessern die Verlässlichkeit der Datenanalyse. Die Integration mehrerer Algorithmen bietet vorzugsweise hohe Flexibilität und Anpassungsfähigkeit an unterschiedliche Szenarien.

In einem weiteren Aspekt wird vorgeschlagen, dass der Bildverarbeitungsalgorithmus ein maschinelles Lernmodell zur Bildverarbeitung, insbesondere ein neuronales Faltungsnetzwerk, aufweist.

Neuronale Faltungsnetzwerke (CNNs) sind besonders effektiv für die Bildverarbeitung und können Muster und Objekte in den Bilddaten erkennen. Alternativ könnten auch Transformer-Modelle und/oder hybride Ansätze verwendet werden. Das maschinelle Lernmodell erhöht vorzugsweise die Genauigkeit und Geschwindigkeit der Bildverarbeitung. Die Verwendung von CNNs ermöglicht vorzugsweise eine effiziente Verarbeitung großer Datenmengen und fördert die Automatisierung.

Allgemein kann der Bildverarbeitungsalgorithmus ein maschinelles Lernmodell und/oder ein statistisches Modell und/oder ein analytisches Modell, insbesondere ein hybrides Modell, umfassend mehrere der vorgenannten Modelle in Kombination, aufweisen.

In einem weiteren Aspekt wird vorgeschlagen, dass das maschinelle Labelgenerierung-Lernmodell unter Verwendung von Pflegeprotokollen, einer Pflegehistorie, der Pflege des Patienten und damit verbundenen Bilddaten trainiert oder zumindest feinabgestimmt wird.

Das Training des Modells auf spezifische und/oder vorbestimmte Pflegeprotokolle ermöglicht vorzugsweise eine anwendungsspezifische Anpassung. Alternativ könnten öffentlich verfügbare oder synthetische Daten verwendet werden, um das Modell zu initialisieren. Das Training auf spezifische Daten erhöht vorzugsweise die Genauigkeit und Kontextsensitivität. Eine Feinabstimmung erlaubt eine personalisierte und kontextabhängige Dokumentation.

In einem weiteren Aspekt wird vorgeschlagen, dass eine Ausgabe der generierten Text- und/oder Sprachlabels als, insbesondere editierbare, Audio- und/oder Textdatei erfolgt.

Die Ausgabe in verschiedenen Formaten ermöglicht vorzugsweise eine einfache Integration in bestehende Systeme. Alternativ können visuelle Darstellungen der Labels in Dashboards erfolgen. Eine solche Flexibilität bei der Ausgabe fördert vorzugsweise die Nutzbarkeit und Anpassungsfähigkeit. Die editierbare Ausgabe erleichtert vorzugsweise Korrekturen und/oder Anpassungen.

In einem weiteren Aspekt wird vorgeschlagen, dass fehlerhafte Labels in einer Revision der Pflegeprotokolle identifiziert und nachfolgend dazu verwendet werden, das maschinelle Labelgenerierung-Lernmodell und/oder den Bildverarbeitungsalgorithmus nachzutrainieren.

Die kontinuierliche Verbesserung des Modells durch fehlerhafte Labels fördert vorzugsweise die Lernfähigkeit. Alternativ könnten externe Feedbackquellen einbezogen werden. Nachtraining erhöht die langfristige Genauigkeit und Robustheit. Das System bleibt adaptiv und passt sich veränderten Bedingungen an.

In einem weiteren Aspekt wird vorgeschlagen, dass ein Computerprogrammprodukt umfassend Befehle enthält, die bei der Ausführung des Programms durch einen Computer diesen veranlassen, die Schritte des vorliegenden Verfahrens nach einer beliebigen Ausführungsform auszuführen.

Das Computerprogrammprodukt bildet die Grundlage für die Implementierung der Verfahren. Die Verfügbarkeit als Softwareprodukt erleichtert die Verbreitung und Integration. Ein einfacher Einsatz auf vorhandener Hardware wird ermöglicht.

Ein Computerprogramm, das die Schritte eines Verfahrens zur automatisierten Dokumentation von Pflegemaßnahmen umsetzt, kann vorzugsweise modular aufgebaut sein und aus mehreren Komponenten bestehen, die vorzugsweise spezifische Aufgaben übernehmen. Es könnte beispielsweise aus den folgenden Modulen bestehen:
Ein Eingabemodul dient vorzugsweise der Integration der Bildaufnahmeeinheit, die entweder tragbar, stationär oder beweglich ist. Dieses Eingabemodul steuert vorzugsweise die Erfassung der Bilddaten, synchronisiert die Aufnahmen bei Bedarf mit anderen Datensätzen (z. B. Zeitstempeln oder Patienteninformationen) und/oder bereitet die Bilddaten für die Verarbeitung vor. Zusätzlich kann dieses Eingabemodul vorzugsweise Filter anwenden, um die Bildqualität zu optimieren, und datenschutzfreundliche Techniken wie Anonymisierung oder Unschärfe von Gesichtsdaten integrieren.

Ein Bildverarbeitungsmodul analysiert vorzugsweise die erfassten Bilddaten mithilfe eines oder mehrerer Bildverarbeitungsalgorithmen. Hierbei könnten neuronale Netzwerke wie Convolutional Neural Networks (CNNs) oder Vision Transformers (ViTs) zur Objekterkennung, Segmentierung, Bewegungsanalyse oder Aktivitätserkennung eingesetzt werden. Dieses Bildverarbeitungsmodul ist vorzugsweise darauf ausgelegt, relevante Informationen wie Handlungen der Pflegekraft, den Zustand des Patienten oder den Einsatz von Pflegeutensilien zu extrahieren.

Ein Labelgenerierungsmodul verarbeitet vorzugsweise die Ergebnisse des Bildverarbeitungsmoduls und erstellt Text- und/oder Sprachlabels. Dieses Labelgenerierungsmodul nutzt vorzugsweise ein maschinelles Lernmodell, wie ein Transformer-Modell, das auf annotierten Pflegeprotokollen und Bilddaten trainiert wurde. Die Labels werden vorzugsweise in strukturierter Form erstellt und können nach Bedarf als Text- oder Audioausgabe formatiert werden. Weiterhin kann das Labelgenerierungsmodul vorzugsweise eine Feedback-Komponente beinhalten, die fehlerhafte Labels identifiziert und diese zur Optimierung des maschinellen Lernmodells nutzt.

Ein Ausgabemodul sorgt vorzugsweise für die Speicherung und Ausgabe der generierten Labels. Es könnte die Labels in verschiedenen Formaten bereitstellen, wie z. B. als editierbare Textdateien, Audioformate oder in Form einer visuellen Benutzeroberfläche, etwa einem Dashboard, das Echtzeit-Updates anzeigt. Dieses Ausgabemodul kann auch eine Schnittstelle zur Integration mit externen Systemen, wie elektronischen Patientenakten (EPA), bereitstellen.

Ein Trainings- und Anpassungsmodul ermöglicht vorzugsweise die Weiterentwicklung und Feinabstimmung der genutzten Modelle. Es könnte ein Nachtraining auf Basis neuer Daten oder fehlerhafter Labels durchführen und so die Präzision und Robustheit des Systems kontinuierlich verbessern. Dabei könnte es sowohl online (während der Nutzung) als auch offline (auf vorbereiteten Datensätzen) arbeiten.

Ein Sicherheitsmodul stellt vorzugsweise sicher, dass die Datenschutzanforderungen eingehalten werden. Es könnte Verschlüsselungstechniken zur Sicherung der gespeicherten Daten einsetzen und den Zugriff auf die Daten auf autorisierte Nutzer beschränken. Dieses Modul kann zudem Mechanismen zur Anonymisierung von sensiblen Informationen wie Gesichtern oder persönlichen Details des Patienten integrieren.

In einem weiteren Aspekt wird vorgeschlagen, dass ein computerlesbarer Datenträger das vorliegende Computerprogrammprodukt speichert.

Der Datenträger dient vorzugsweise der langfristigen Speicherung und Verteilung des Programms. Die Speicherung auf Datenträgern sichert vorzugsweise die Portabilität. Flexible Verteilung und Sicherung werden ermöglicht.

Ein computerlesbarer Datenträger, auf dem ein solches Computerprogramm gespeichert ist, kann in vielfältiger Weise ausgebildet sein. Ein typischer Datenträger könnte ein physisches Medium wie eine CD, DVD oder Blu-ray-Disc sein, auf der das Programm dauerhaft gespeichert wird. Alternativ könnte ein Flash-basiertes Speichermedium wie ein USB-Stick oder eine SSD genutzt werden, das eine größere Speicherkapazität und einfachere Handhabung bietet.

Ein weiterer Ansatz wäre ein Cloud-basierter Datenträger, bei dem die Programmdaten auf einem entfernten Server gespeichert sind und über das Internet aufgerufen werden können. Solche Lösungen sind besonders geeignet für Szenarien, in denen Updates und gemeinsamer Zugriff auf das Programm erforderlich sind. In allen Fällen könnte der Datenträger zusätzliche Sicherheitsmechanismen wie Passwortschutz oder Verschlüsselung bieten, um unbefugten Zugriff zu verhindern.

Der Datenträger kann zudem so gestaltet sein, dass er direkt mit bestehenden Systemen, wie Krankenhausinformationssystemen, kompatibel ist und die nahtlose Integration ermöglicht. Dies fördert die einfache Verteilung und Skalierbarkeit des Programms in verschiedenen Pflegeumgebungen.

Das Training des maschinellen Lernmodells für das vorliegende Verfahren kann vorzugsweise in mehreren Phasen erfolgen, um die Anforderungen der automatisierten Pflegedokumentation flexibel zu erfüllen. Zunächst wird vorzugsweise ein Basismodell des maschinellen Lernmodells bereitgestellt, das auf allgemeinen Daten trainiert wird, bevor es durch spezifische Pflegedaten feinabgestimmt wird. Dieser Prozess umfasst vorzugsweise die Phasen der Datensammlung, Datenvorbereitung, Modelltraining, Validierung und Optimierung, um eine hohe Genauigkeit und Robustheit zu gewährleisten.

Die Trainingsdaten für das maschinelle Lernmodell können vorzugsweise aus einer Vielzahl von Quellen stammen, wie Video- und Bildaufnahmen von realen Pflegeinteraktionen, annotierten Pflegeprotokollen oder synthetisch generierten Daten. Diese Daten könnten unterschiedliche Pflegehandlungen umfassen, wie Körperpflege, Mobilisation, Medikamentengabe oder Wundversorgung, und könnten durch Metadaten wie Zeitstempel, Pflegeumgebungsmerkmale oder Sensorinformationen (z. B. Tiefendaten) ergänzt werden. Die Trainingsdaten können vorzugsweise in Formaten wie MP4 oder AVI für Videodaten, JPEG oder PNG für Bilder und CSV oder JSON für zugehörige Annotationen vorliegen. Annotationen könnten dabei Labels enthalten, die den Daten semantische Bedeutungen wie "Mobilisation des Patienten" oder "Verabreichung von Medikamenten" zuweisen.

Die Verarbeitung der Trainingsdaten durch das maschinelle Lernmodell erfolgt vorzugsweise durch eine Vorverarbeitung, die die Normalisierung der Bilddaten (z. B. Anpassung von Auflösung und Farbwerten), die Extraktion relevanter Merkmale (z. B. Bewegungsmuster oder Objektkonturen) und die Segmentierung der Szenen umfasst. Die annotierten Labels dienen vorzugsweise als Zielwerte für das Training des Modells. Während des Trainingsprozesses abstrahiert das Modell die Merkmale schrittweise, um die Beziehungen zwischen den Eingaben (Bilddaten) und den Ausgaben (Labels) zu erlernen.

Ein Foundation-Modellansatz könnte vorzugsweise zur Effizienzsteigerung genutzt werden. Dabei wird ein großes vortrainiertes Modell eingesetzt, das vorzugsweise auf umfangreichen, allgemeinen Datensätzen wie großen Video- oder Bilddatenbanken trainiert wurde. Dieses Foundation-Modell besitzt vorzugsweise ein breites Verständnis von allgemeinen visuellen und semantischen Mustern und reduziert die Notwendigkeit für umfangreiche Pflege-spezifische Daten. Anschließend erfolgt vorzugsweise eine Feinabstimmung (Fine-Tuning) des Foundation-Modells mit domänenspezifischen Pflege-Daten, wie annotierten Videos aus Pflegeheimen oder Krankenhäusern, um die spezifischen Anforderungen der Pflegedokumentation abzudecken.

Ein Anwendungsbeispiel des vorliegenden Verfahrens könnte vorzugsweise in einem Krankenhaus für die Dokumentation von Mobilisationsmaßnahmen bei bettlägerigen Patienten umgesetzt werden. In einem solchen Szenario könnte eine stationär installierte Kamera im Patientenzimmer vorzugsweise die Interaktionen zwischen der Pflegekraft und dem Patienten aufzeichnen. Das System erfasst vorzugsweise Szenen wie das Umlagern des Patienten im Bett, das Aufsetzen oder den Transfer in einen Rollstuhl. Die Kamera könnte vorzugsweise die Daten an das Bildverarbeitungsmodul übermitteln, das die Szenen in Echtzeit analysiert und relevante Bewegungsmuster erkennt. Ein maschinelles Lernmodell, das vorzugsweise speziell auf Mobilisationsdaten trainiert wurde, könnte daraufhin Text- und/oder Sprachlabels wie "Umlagerung des Patienten abgeschlossen" oder "Patient in Rollstuhl transferiert" generieren.

Die generierten Labels könnten vorzugsweise automatisch in das elektronische Patientenakten-System des Krankenhauses integriert werden. Gleichzeitig könnte eine Dashboard-Schnittstelle den Pflegekräften ermöglichen, die Dokumentation in Echtzeit zu überprüfen und gegebenenfalls zu bearbeiten. Fehlerhafte Labels könnten vorzugsweise manuell korrigiert werden, wobei diese Korrekturen vorzugsweise in die Feedback-Schleife des maschinellen Lernmodells einfließen, um das maschinelle Lernmodell kontinuierlich zu optimieren. Dieses Beispiel zeigt, wie das Verfahren vorzugsweise effizient eingesetzt werden kann, um den Dokumentationsaufwand zu reduzieren, die Genauigkeit zu erhöhen und die Pflegekräfte zu entlasten.

Das maschinelle Lernmodell kann durch die Verwendung von zusätzlichen Audiodaten, die vorzugsweise mittels eines Mikrofons erfasst werden, weiter verbessert werden, da diese eine zusätzliche Dimension der Kontextinformationen liefern. Audiodaten könnten vorzugsweise Sprache, Umgebungsgeräusche und/oder spezifische akustische Ereignisse wie das Rascheln von Kleidung, das Öffnen von Verpackungen und/oder das Geräusch eines Rollstuhls umfassen. Diese Informationen können vorzugsweise durch ein Mikrofon erfasst werden, das entweder in die Bildaufnahmeeinheit integriert ist, wie bei einer Smart-Brille oder einer stationären Kamera, oder als separates Gerät genutzt wird. Die Synchronisation von Audio- und Bilddaten könnte vorzugsweise zeitliche Übereinstimmungen zwischen visuellen und akustischen Signalen herstellen. So könnte das maschinelle Lernmodell durch das Hören eines Reißverschlussgeräuschs in Kombination mit einer visuellen Handlung vorzugsweise erkennen, dass ein Kleidungsstück angezogen wird.

Spracherkennung und/oder -analyse könnten vorzugsweise in das System integriert werden, um gesprochene Worte oder Sätze während der Pflege zu transkribieren. Dies kann vorzugsweise Hinweise auf die durchgeführte Handlung liefern, wie etwa, wenn die Pflegekraft sagt: "Ich messe jetzt Ihren Blutdruck." Solche sprachlichen Hinweise könnten vorzugsweise die visuelle Analyse ergänzen und das Modell bei der korrekten Interpretation der Handlung unterstützen. Darüber hinaus könnten Umgebungsgeräusche wie das Klicken eines Blutdruckmessgeräts, das Zischen einer Sauerstoffmaske oder das Rauschen von Wasser analysiert werden. Diese Geräusche könnten vorzugsweise als Indikatoren für bestimmte Pflegehandlungen dienen, etwa das Händewaschen oder das Anlegen von Geräten.

Die Kombination von Bild- und Audiodaten durch multimodale Datenfusion kann vorzugsweise eine ganzheitliche Analyse der Pflegehandlungen ermöglichen. Transformer-Modelle oder andere spezialisierte Architekturen für multimodale Daten könnten vorzugsweise eingesetzt werden, um visuelle und akustische Informationen zusammenzuführen. Diese Fusion erlaubt es dem Modell vorzugsweise, unklare Informationen aus einem Datenstrom durch den anderen zu ergänzen. Trainingsdaten könnten vorzugsweise um annotierte Audioclips erweitert werden, die beispielsweise typische Geräusche und Sprachmuster während der Pflege enthalten. Dadurch wird das Modell vorzugsweise für akustische Variationen wie unterschiedliche Tonlagen und/oder Dialekte sensibilisiert.

Ein praktisches Beispiel zeigt vorzugsweise die Vorteile dieser Integration. Angenommen, eine Pflegekraft misst den Blutdruck eines Patienten. Das visuelle Modell könnte vorzugsweise die Pflegekraft und das Blutdruckmessgerät erkennen, wäre jedoch möglicherweise nicht in der Lage, die spezifische Handlung eindeutig zu klassifizieren. Durch die Integration von Audiodaten könnte das Modell vorzugsweise das Zischen der Luft beim Aufpumpen des Geräts sowie die Aussage der Pflegekraft "Ich überprüfe jetzt Ihren Blutdruck" hören. Diese Informationen ermöglichen es dem Modell vorzugsweise, das Label "Blutdruckmessung durchgeführt" mit hoher Präzision zu generieren.

Durch diese Integration von Audiodaten könnte das maschinelle Lernmodell vorzugsweise präziser und flexibler werden, insbesondere in Situationen, in denen die Sicht auf die Handlung eingeschränkt ist und/oder visuelle Daten allein nicht ausreichen. Die Kombination von visuellen und akustischen Signalen verbessert vorzugsweise die Kontextualisierung und ermöglicht eine schnellere, zuverlässigere und umfassendere automatische Dokumentation von Pflegehandlungen.

In einem weiteren Aspekt kann die Datensicherheit und Anonymität des Patienten vorzugsweise durch eine Reihe technischer Maßnahmen gewährleistet werden. Die erfassten Bilddaten von Interaktionen zwischen einer Pflegekraft und einem Patienten können vorzugsweise vor der Verarbeitung durch den Bildverarbeitungsalgorithmus anonymisiert werden. Hierbei könnten vorzugsweise Verfahren wie Gesichtserkennung und -maskierung zum Einsatz kommen, um Gesichter und/oder andere identifizierbare Merkmale automatisch zu erkennen und durch Unschärfe, Pixelation oder vollständige Abdeckung unkenntlich zu machen.

Um unbefugten Zugriff zu verhindern, könnten die Bilddaten vorzugsweise während der Übertragung von der Bildaufnahmeeinheit an die Auswerte- und Recheneinheit durch Ende-zu-Ende-Verschlüsselung gesichert werden. Ein Verschlüsselungsverfahren wie AES-256 könnte vorzugsweise verwendet werden, um sicherzustellen, dass nur autorisierte Systeme die Daten entschlüsseln und weiterverarbeiten können. Ergänzend könnten pseudonymisierte Datenstrukturen genutzt werden, bei denen persönliche Identifikatoren, wie der Name des Patienten, durch eindeutige, nicht rückverfolgbare Codes ersetzt werden.

Die Speicherung der Daten könnte vorzugsweise auf lokalen, geschützten Servern erfolgen, die physisch und digital gegen Angriffe abgesichert sind. Alternativ könnten vorzugsweise Edge-Computing-Lösungen eingesetzt werden, bei denen die Verarbeitung und Anonymisierung der Daten bereits auf der Bildaufnahmeeinheit oder in einer lokalen Einheit stattfinden, sodass keine sensiblen Daten in externe Netzwerke übertragen werden müssen. Alternativ kann auch ein Löschen der erfassten Daten nach Visierung des Berichts erfolgen.

Zur Sicherstellung der Datensicherheit könnten vorzugsweise Zugriffskontrollsysteme implementiert werden, die nur autorisierten Personen den Zugang zu bestimmten Datenbereichen erlauben. Diese Systeme könnten vorzugsweise auf Zwei-Faktor-Authentifizierung (2FA) und/oder biometrischen Verfahren basieren. Zusätzlich könnten vorzugsweise alle Datenoperationen durch ein Audit-Logging-System protokolliert werden, um verdächtige Zugriffsversuche zu identifizieren und gegebenenfalls zu blockieren.

Schließlich könnte ein datenschutzorientierter Ansatz durch den Einsatz von differenzieller Privatsphäre ergänzt werden, bei dem Rauschsignale zu den Daten hinzugefügt werden, um Rückschlüsse auf individuelle Patienten zu verhindern, während die nützliche Information für die Verarbeitung erhalten bleibt. Diese technischen Maßnahmen könnten vorzugsweise eine sichere und anonymisierte Verarbeitung der Daten im Rahmen des beanspruchten Verfahrens gewährleisten.

Die beschriebenen Ausgestaltungen und Weiterbildungen lassen sich beliebig miteinander kombinieren.

Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung.

### Kurze Beschreibung der Zeichnungen

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung.

Andere Ausführungsformen und viele der genannten Vorteile ergeben sich im Hinblick auf die Zeichnungen. Die dargestellten Elemente der Zeichnungen sind nicht notwendigerweise maßstabsgetreu zueinander gezeigt.
- Fig. 1: zeigt ein schematisches Flussdiagramm eines Ausführungsbeispiels des Verfahrens.
- Fig. 2: zeigt eine schematische Ansicht eines Blockdiagramms zu der vorliegenden Vorrichtung.
- Fig. 3: zeigt eine schematische Ansicht eines Blockdiagramms zu der vorliegenden Vorrichtung.

### Detaillierte Beschreibung der Zeichnungen

In den Figuren der Zeichnungen bezeichnen gleiche Bezugszeichen gleiche oder funktionsgleiche Elemente, Bauteile oder Komponenten, soweit nichts Gegenteiliges angegeben ist.

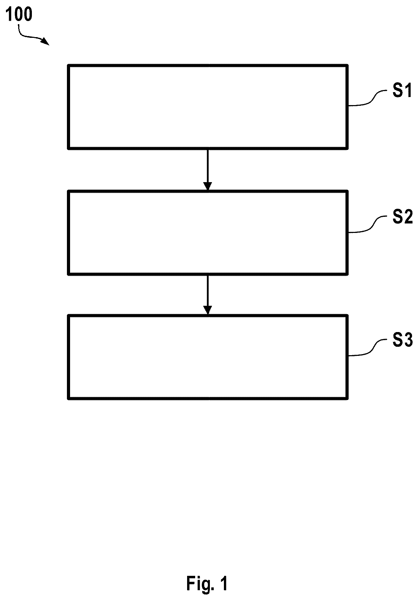

Fig. 1 zeigt ein schematisches Flussdiagramm eines Ausführungsbeispiels eines vorliegenden Verfahrens zur automatisierten Dokumentation von Pflegemaßnahmen zur Pflege eines Patienten.

Das Verfahren kann in einer beliebigen Ausführungsform zumindest teilweise durch eine Vorrichtung 100 ausgeführt werden, die hierzu mehrere nicht näher dargestellte Komponenten, beispielsweise eine oder mehrere Bereitstellungseinrichtungen und/oder mindestens eine Auswerte- und Recheneinrichtung umfassen kann. Es versteht sich, dass die Bereitstellungseinrichtung gemeinsam mit der Auswerte- und Recheneinrichtung ausgebildet sein kann, oder von dieser unterschiedlich sein kann. Ferner kann die Vorrichtung 100, die ein Teil eines Systems sein kann, eine Speichereinrichtung und/oder eine Ausgabeeinrichtung und/oder eine Anzeigeeinrichtung und/oder eine Eingabeeinrichtung umfassen.

Das computerimplementierte Verfahren umfasst mindestens die folgenden Schritte:
In einem Schritt S1 erfolgt ein Erfassen von Bilddaten von Interaktionen zwischen einer Pflegekraft und einem Patienten unter Verwendung einer Bildaufnahmeeinheit.

In einem Schritt S2 erfolgt ein Verarbeiten der erfassten Bilddaten unter Verwendung eines Bildverarbeitungsalgorithmus zum Erkennen der Interaktionen in den Bilddaten.

In einem Schritt S3 erfolgt ein Generieren von Text- und/oder Sprachlabels zu den erkannten Interaktionen unter Verwendung eines maschinellen Labelgenerierung-Lernmodells zur automatisierten Dokumentation von Pflegemaßnahmen unter Verwendung der generierten Text- und/oder Sprachlabels.

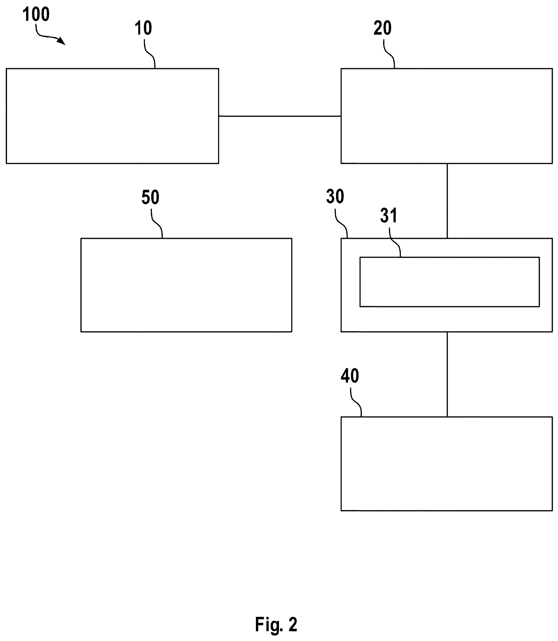

Fig. 2 zeigt eine schematische Darstellung der Architektur der Vorrichtung 100 zur automatisierten Dokumentation von Pflegemaßnahmen. Die Vorrichtung 100 umfasst eine Bildaufnahmeeinheit 10, die visuellen Daten von Interaktionen 130 zwischen einer Pflegekraft 110 und einem Patienten 120 erfasst. Die Bildaufnahmeeinheit 10 kann verschiedene Ausführungen umfassen, wie eine stationär installierte Kamera, eine tragbare Kamera oder eine in eine (Smart-) Brille integrierte Kamera. Die Bildaufnahmeeinheit 10 kann auch an einem Kopf der Pflegekraft 110 angeordnet sein. Die erfassten Daten werden vorzugsweise über eine Schnittstelle an eine Auswerte- und Recheneinheit 20 weitergeleitet.

Die Auswerte- und Recheneinheit 20 übernimmt die Analyse der Bilddaten und umfasst vorzugsweise verschiedene Module. Zunächst erfolgt die Verarbeitung der Daten durch ein Bildverarbeitungsmodul 25, das visuelle Merkmale extrahiert, wie beispielsweise die Position der Pflegekraft 110, des Patienten 120 und etwaiger Pflegeutensilien. Die verarbeiteten Bilddaten werden anschließend im Labelgenerierungsmodul 30 analysiert, das mithilfe eines maschinellen Lernmodells 31 automatisch Text- und/oder Sprachlabels erzeugt. Diese Labels beschreiben die identifizierten Pflegehandlungen, wie beispielsweise "Patient mobilisiert" oder "Blutdruckmessung durchgeführt".

Das Labelgenerierungsmodul 30 gibt die erzeugten Labels vorzugsweise an ein Ausgabemodul 40 weiter, das die Ausgabe vorzugsweise in unterschiedlichen Formaten bereitstellt. Das Ausgabemodul 40 ermöglicht vorzugsweise die Generierung von Textdokumenten, die direkt in elektronische Patientenakten integriert werden können, und/oder die Ausgabe von Sprachinformationen zur Unterstützung der Pflegekraft. Eine, insbesondere zentrale, Prozesseinheit 50 steuert vorzugsweise den gesamten Datenfluss zwischen den einzelnen Modulen und stellt vorzugsweise sicher, dass die Daten konsistent verarbeitet und weitergeleitet werden. Die Architektur ermöglicht eine automatisierte, präzise und effiziente Dokumentation von Pflegehandlungen.

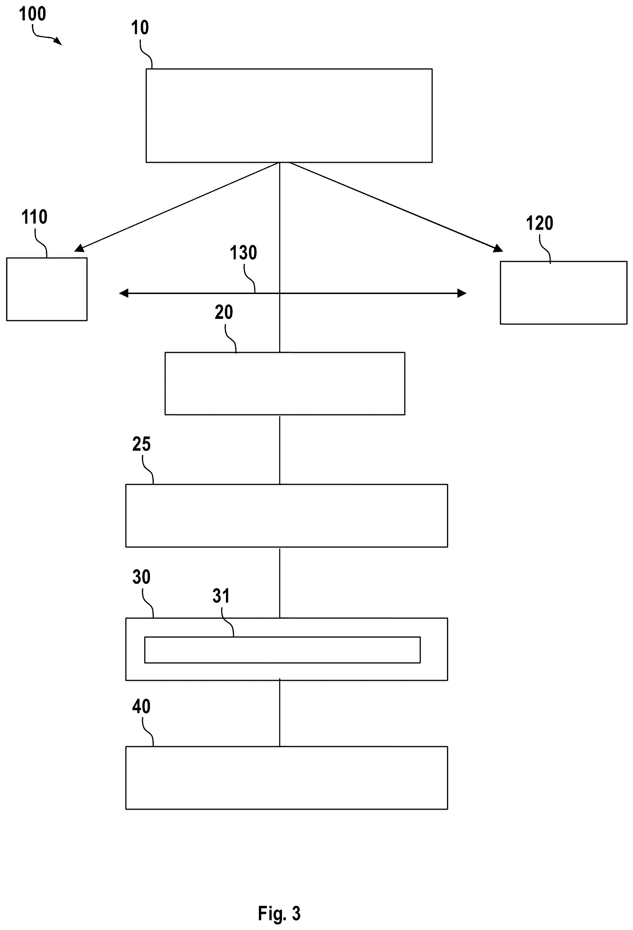

Figur 3 zeigt die räumliche Anordnung der einzelnen Systemkomponenten in einem typischen Pflegeumfeld. In der Mitte der Darstellung ist ein Patient 120 schematisch zu sehen, der von einer Pflegekraft 110 betreut wird. Die Interaktionen 130 zwischen der Pflegekraft 110 und dem Patienten 120 werden durch eine Bildaufnahmeeinheit 10 erfasst. Diese Bildaufnahmeeinheit 10 kann als tragbare Komponente, beispielsweise in Form einer Smart-Brille, oder als stationär installierte Kamera in einem Patientenzimmer ausgeführt sein.

Die Bildaufnahmeeinheit 10 zeichnet die visuellen Daten der Interaktionen 130 auf und synchronisiert diese vorzugsweise mit zusätzlichen Informationen, wie Bewegungs- oder Umgebungsdaten. Die erfassten Daten werden an die Auswerte- und Recheneinheit 20 übermittelt, die beispielsweise Bewegungen, Objekte und/oder Handlungen analysiert. Beispielsweise kann erkannt werden, ob die Pflegekraft 110 den Patienten 120 umlagert, ein Medikament verabreicht und/oder Vitalparameter misst.

Nach der Analyse der Interaktionen 130 in der Auswerte- und Recheneinheit 20 werden die Ergebnisse vorzugsweise an das Labelgenerierungsmodul 30 weitergeleitet. Das Labelgenerierungsmodul 30 nutzt das mindestens eine maschinelle Lernmodell oder eine Modellkomposition aus mehreren maschinellen und/oder statistischen und/oder analytischen Modellen, um die Daten in, insbesondere aussagekräftige, Labels zur Dokumentation der Pflege des Patienten 120 zu übersetzen. Diese Labels könnten beispielsweise "Patient gewaschen", "Verbandswechsel durchgeführt" oder "Medikament gegeben" lauten. Die Labels werden vorzugsweise automatisch im Kontext der Pflegehandlung erstellt und an das Ausgabemodul 40 übermittelt. Das Ausgabemodul 40 gibt dann vorzugsweise die Pflege-Dokumentation unter Verwendung der generierten Labels in strukturierter Form aus. Dabei können vorzugsweise auch noch allgemeine Kontextinformationen und/oder Vorlage-Informationen zu dem Aufbau und/oder der Art der Dokumentation auf Basis von Sprachverarbeitung und/oder unter Verwendung eines Sprachmodells erzeugt werden. Diese Ausgabe erfolgt entweder als Textdokument, das beispielsweise in einer digitalen Patientenakte gespeichert wird, und/oder als Sprachdatei, die Pflegekräfte auditiv unterstützt.

### Bezugszeichenliste

- 10: Bildaufnahmeeinheit
- 20: Auswerte- und Recheneinheit
- 25: Bildverarbeitungsmodul
- 30: Labelgenerierungsmodul
- 31: Maschinelles Lernmodell
- 40: Ausgabemodul
- 50: Prozesseinheit
- 100: Vorrichtung
- 110: Pflegekraft
- 120: Patient
- 130: Interaktionen
- S1: Schritt 1 (Erfassen von Bilddaten)
- S2: Schritt 2 (Verarbeiten von Bilddaten)
- S3: Schritt 3 (Generieren von Labels)

## Patentansprüche

1. Verfahren zur automatisierten Dokumentation von Pflegemaßnahmen zur Pflege eines Patienten (120), aufweisend die Schritte:
- Erfassen (S1) von Bilddaten von Interaktionen (130) zwischen einer Pflegekraft (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen in den Bilddaten; und
- Generieren (S3) von Text- und/oder Sprachlabels zu den erkannten Interaktionen (130) unter Verwendung eines maschinellen Labelgenerierung-Lernmodells (31) zur automatisierten Dokumentation von Pflegemaßnahmen unter Verwendung der generierten Text- und/oder Sprachlabels.

2. Verfahren nach Anspruch 1, wobei die Bildaufnahmeeinheit (10) eine Kamera und/oder eine Smart-Brille und/oder ein anderes tragbares Gerät zur Bilderfassung aufweist.

3. Verfahren nach Anspruch 1 oder 2, wobei die Bildaufnahmeeinheit (10) in einem Raum, in dem der Patient (120) gepflegt wird, und/oder an einem Körper der Pflegekraft (110) und/oder an einem Körper des Patienten (120) angeordnet ist.

4. Verfahren nach einem der Ansprüche 1 bis 3, wobei der Bildverarbeitungsalgorithmus eine Segmentierung der Bilddaten und/oder eine Objekterkennung und/oder eine Bewegungsanalyse und/oder eine Aktivitätserkennung und/oder eine Gesichtserkennung und/oder eine Anomalieerkennung und/oder eine Objektverfolgung und/oder eine Klassifikation von Handlungen und/oder eine multimodale Bildverarbeitung und/oder eine Feature-Extraktion aufweist.

5. Verfahren nach einem der Ansprüche 1 bis 4, wobei Bildverarbeitungsalgorithmus ein maschinelles Lernmodell zur Bildverarbeitung aufweist.

6. Verfahren nach einem der Ansprüche 1 bis 5, wobei das maschinelle Labelgenerierung-Lernmodell unter Verwendung von Pflegeprotokollen einer Pflegehistorie der Pflege des Patienten (120) und damit verbundenen Bilddaten trainiert wird oder zumindest feinabgestimmt wird.

7. Verfahren nach einem der Ansprüche 1 bis 6, wobei eine Ausgabe der generierten Text- und/oder Sprachlabels als Audio- und/oder Textdatei erfolgt.

8. Verfahren nach einem der Ansprüche 1 bis 7, wobei fehlerhafte Labels in einer Revision der Pflegeprotokolle identifiziert und nachfolgend dazu verwendet werden, das maschinelle Labelgenerierung-Lernmodell und/oder den Bildverarbeitungsalgorithmus nachzutrainieren.

9. Computerprogrammprodukt, umfassend Befehle, die bei der Ausführung des Programms durch einen Computer diesen veranlassen, die Schritte des Verfahrens nach einem der Ansprüche 1 bis 8 auszuführen.

10. Vorrichtung (100) zur automatisierten Dokumentation von Pflegemaßnahmen zur Pflege eines Patienten, wobei die Vorrichtung (100) eine Auswerte- und Recheneinrichtung (20) aufweist, die dazu ausgebildet ist, die folgenden Schritte auszuführen:
- Erfassen (S1) von Bilddaten von Interaktionen (130) zwischen einer Pflegekraft (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen (130) in den Bilddaten; und
- Generieren (S3) von Text- und/oder Sprachlabels zu den erkannten Interaktionen (130) unter Verwendung eines maschinellen Lernmodells (31).

## Geänderte Patentansprüche

### Geänderte Patentansprüche gemäss Regel 137(2) EPÜ.

**1.** Verfahren zur automatisierten Dokumentation von Pflegemaßnahmen zur Pflege eines Patienten (120), aufweisend die Schritte:
- Erfassen (S1) von Bilddaten von Interaktionen (130) zwischen einer Pflegekraft (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen in den Bilddaten; und
- Generieren (S3) von Text- und/oder Sprachlabels zu den erkannten Interaktionen (130) unter Verwendung eines maschinellen Labelgenerierung-Lernmodells (31) zur automatisierten Dokumentation von Pflegemaßnahmen unter Verwendung der generierten Text- und/oder Sprachlabels,
**dadurch gekennzeichnet, dass** zusätzlich zu den Bilddaten Audiodaten der Interaktionen erfasst und gemeinsam mit den Bilddaten verarbeitet werden, und das maschinelle Labelgenerierung-Lernmodell (31) die Text- und/oder Sprachlabels unter Verwendung einer multimodalen Analyse der Bild- und Audiodaten kontextabhängig generiert.

**2.** Verfahren nach Anspruch 1, wobei die Bildaufnahmeeinheit (10) eine Kamera und/oder eine Smart-Brille und/oder ein anderes tragbares Gerät zur Bilderfassung aufweist.

**3.** Verfahren nach Anspruch 1 oder 2, wobei die Bildaufnahmeeinheit (10) in einem Raum, in dem der Patient (120) gepflegt wird, und/oder an einem Körper der Pflegekraft (110) und/oder an einem Körper des Patienten (120) angeordnet ist.

**4.** Verfahren nach einem der Ansprüche 1 bis 3, wobei der Bildverarbeitungsalgorithmus eine Segmentierung der Bilddaten und/oder eine Objekterkennung und/oder eine Bewegungsanalyse und/oder eine Aktivitätserkennung und/oder eine Gesichtserkennung und/oder eine Anomalieerkennung und/oder eine Objektverfolgung und/oder eine Klassifikation von Handlungen und/oder eine multimodale Bildverarbeitung und/oder eine Feature-Extraktion aufweist.

**5.** Verfahren nach einem der Ansprüche 1 bis 4, wobei Bildverarbeitungsalgorithmus ein maschinelles Lernmodell zur Bildverarbeitung aufweist.

**6.** Verfahren nach einem der Ansprüche 1 bis 5, wobei das maschinelle Labelgenerierung-Lernmodell unter Verwendung von Pflegeprotokollen einer Pflegehistorie der Pflege des Patienten (120) und damit verbundenen Bilddaten trainiert wird oder zumindest feinabgestimmt wird.

**7.** Verfahren nach einem der Ansprüche 1 bis 6, wobei eine Ausgabe der generierten Text- und/oder Sprachlabels als Audio- und/oder Textdatei erfolgt.

**8.** Verfahren nach einem der Ansprüche 1 bis 7, wobei fehlerhafte Labels in einer Revision der Pflegeprotokolle identifiziert und nachfolgend dazu verwendet werden, das maschinelle Labelgenerierung-Lernmodell und/oder den Bildverarbeitungsalgorithmus nachzutrainieren.

**9.** Computerprogrammprodukt, umfassend Befehle, die bei der Ausführung des Programms durch einen Computer diesen veranlassen, die Schritte des Verfahrens nach einem der Ansprüche 1 bis 8 auszuführen.

**10.** Vorrichtung (100) zur automatisierten Dokumentation von Pflegemaßnahmen zur Pflege eines Patienten, wobei die Vorrichtung (100) eine Auswerte- und Recheneinrichtung (20) aufweist, die dazu ausgebildet ist, die folgenden Schritte auszuführen:
- Erfassen (S1) von Bilddaten von Interaktionen (130) zwischen einer Pflegekraft (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen (130) in den Bilddaten; und
- Generieren (S3) von Text- und/oder Sprachlabels zu den erkannten Interaktionen (130) unter Verwendung eines maschinellen Lemmodells (31) **dadurch gekennzeichnet, dass** zusätzlich zu den Bilddaten Audiodaten der Interaktionen erfasst und gemeinsam mit den Bilddaten verarbeitet werden, und das maschinelle Labelgenerierung-Lernmodell (31) die Text- und/oder Sprachlabels unter Verwendung einer multimodalen Analyse der Bild- und Audiodaten kontextabhängig generiert.

**1.** Verfahren zur automatisierten Dokumentation von Pflegemaßnahmen zur Pflege eines Patienten (120), aufweisend die Schritte:
- Erfassen (S1) von Bilddaten von Interaktionen (130) zwischen einer Pflegekraft (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen in den Bilddaten; und
- Generieren (S3) von Text- und/oder Sprachlabels zu den erkannten Interaktionen (130) unter Verwendung eines maschinellen Labelgenerierung-Lernmodells (31) zur automatisierten Dokumentation von Pflegemaßnahmen unter Verwendung der generierten Text- und/oder Sprachlabels,
**dadurch gekennzeichnet, dass** zusätzlich zu den Bilddaten Audiodaten der Interaktionen erfasst und gemeinsam mit den Bilddaten verarbeitet werden, und das maschinelle Labelgenerierung-Lernmodell (31) die Text- und/oder Sprachlabels unter Verwendung einer multimodalen Analyse der Bild- und Audiodaten kontextabhängig generiert, wobei die multimodale Analyse eine gemeinsame Auswertung von Bild- und Audiodaten zur Kontextinterpretation der Interaktionen umfasst.

**2.** Verfahren nach Anspruch 1, wobei die Bildaufnahmeeinheit (10) eine Kamera und/oder eine Smart-Brille und/oder ein anderes tragbares Gerät zur Bilderfassung aufweist.

**3.** Verfahren nach Anspruch 1 oder 2, wobei die Bildaufnahmeeinheit (10) in einem Raum, in dem der Patient (120) gepflegt wird, und/oder an einem Körper der Pflegekraft (110) und/oder an einem Körper des Patienten (120) angeordnet ist.

**4.** Verfahren nach einem der Ansprüche 1 bis 3, wobei der Bildverarbeitungsalgorithmus eine Segmentierung der Bilddaten und/oder eine Objekterkennung und/oder eine Bewegungsanalyse und/oder eine Aktivitätserkennung und/oder eine Gesichtserkennung und/oder eine Anomalieerkennung und/oder eine Objektverfolgung und/oder eine Klassifikation von Handlungen und/oder eine multimodale Bildverarbeitung und/oder eine Feature-Extraktion aufweist.

**5.** Verfahren nach einem der Ansprüche 1 bis 4, wobei Bildverarbeitungsalgorithmus ein maschinelles Lernmodell zur Bildverarbeitung aufweist.

**6.** Verfahren nach einem der Ansprüche 1 bis 5, wobei das maschinelle Labelgenerierung-Lernmodell unter Verwendung von Pflegeprotokollen einer Pflegehistorie der Pflege des Patienten (120) und damit verbundenen Bilddaten trainiert wird oder zumindest feinabgestimmt wird.

**7.** Verfahren nach einem der Ansprüche 1 bis 6, wobei eine Ausgabe der generierten Text- und/oder Sprachlabels als Audio- und/oder Textdatei erfolgt.

**8.** Verfahren nach einem der Ansprüche 1 bis 7, wobei fehlerhafte Labels in einer Revision der Pflegeprotokolle identifiziert und nachfolgend dazu verwendet werden, das maschinelle Labelgenerierung-Lernmodell und/oder den Bildverarbeitungsalgorithmus nachzutrainieren.

**9.** Computerprogrammprodukt, umfassend Befehle, die bei der Ausführung des Programms durch einen Computer diesen veranlassen, die Schritte des Verfahrens nach einem der Ansprüche 1 bis 8 auszuführen.

**10.** Vorrichtung (100) zur automatisierten Dokumentation von Pflegemaßnahmen zur Pflege eines Patienten, wobei die Vorrichtung (100) eine Auswerte- und Recheneinrichtung (20) aufweist, die dazu ausgebildet ist, die folgenden Schritte auszuführen:
- Erfassen (S1) von Bilddaten von Interaktionen (130) zwischen einer Pflegekraft (110) und einem Patienten (120) unter Verwendung einer Bildaufnahmeeinheit (10);
- Verarbeiten (S2) der erfassten Bilddaten unter Verwendung eines Bildverarbeitungsalgorithmus zum Erkennen der Interaktionen (130) in den Bilddaten; und
- Generieren (S3) von Text- und/oder Sprachlabels zu den erkannten Interaktionen (130) unter Verwendung eines maschinellen Lemmodells (31) **dadurch gekennzeichnet, dass** zusätzlich zu den Bilddaten Audiodaten der Interaktionen erfasst und gemeinsam mit den Bilddaten verarbeitet werden, und das maschinelle Labelgenerierung-Lernmodell (31) die Text- und/oder Sprachlabels unter Verwendung einer multimodalen Analyse der Bild- und Audiodaten kontextabhängig generiert, wobei die multimodale Analyse eine gemeinsame Auswertung von Bild- und Audiodaten zur Kontextinterpretation der Interaktionen umfasst.
